(19) Europäisches Patentamt
European Patent Office
Office européen des brevets

(11) Numéro de publication : **0 032 077**
**B1**

(12) # FASCICULE DE BREVET EUROPÉEN

(45) Date de publication du fascicule du brevet : **22.06.83**

(51) Int. Cl.$^3$ : **C 07 C143/70**, C 07 C 25/13

(21) Numéro de dépôt : **80401752.3**

(22) Date de dépôt : **08.12.80**

(54) **Procédé de préparation de fluorures de fluorobenzène sulfonyle.**

(30) Priorité : **20.12.79 FR 7931220**

(43) Date de publication de la demande :
**15.07.81 Bulletin 81/28**

(45) Mention de la délivrance du brevet :
**22.06.83 Bulletin 83/25**

(84) Etats contractants désignés :
**AT BE CH DE FR GB IT LI LU NL SE**

(56) Documents cités :
**FR A 1 360 917**
**FR A 1 446 914**
**CHEMICAL ABSTRACTS, vol. 64, no. 6, 14 mars 1966, colonne 8059a COLUMBUS OHIO (US) & Ind. Eng. Chem. 58 (1) 48-55 (1966) A. K. BARBOUR et al. : « Highly fluorinated aromatic and alicyclic compounds »**
**CHEMICAL ABSTRACTS, vol. 63, no. 11, 22 novembre 1965, colonne 14740e COLUMBUS OHIO (US) & Zh. Vses. Khim. Obschchestva im D. I. Mendeleeva 10 (4), 466-7 (1965) G. G. YAKOBSON et al. : « Reaction of aromatic sulfonyl chlorides with potassium fluoride »**
**CHEMICAL ABSTRACTS, vol. 66, no. 21, 22 mai 1967, réf. no. 94741e, page 8855 COLUMBUS OHIO (US) & Zh. Obshch. Khim. 37 (1), 163-70 (1967) G. G. YAKOBSON et al. : « Aromatic fluoro derivatives. XXIV. Fluorine-substituted benzenesulfonyl fluorides »**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Soula, Gérard**
**33, rue Nungesser**
**F-69330 - Meyzieu (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 032 077 B1

Procédé de préparation de fluorures de fluorobenzène sulfonyle

La présente invention a pour objet un procédé de préparation de fluorures de fluorobenzène sulfonyle ; elle concerne plus particulièrement la préparation de fluorures de fluorobenzène sulfonyle par échange d'au moins un atome de chlore par un atome de fluor sur un fluorure de chlorobenzène sulfonyle.

On entend au sens de la présente invention par fluorure de chlorobenzène sulfonyle, un composé de formule générale :

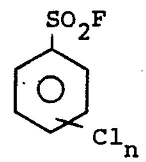

(I)

dans laquelle n est compris entre 1 et 5 ($1 \leq n \leq 5$). Les composés préparés par le procédé selon l'invention ont pour formule générale :

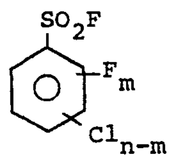

(II)

dans laquelle les atomes de fluor sont en ortho et/ou en para du groupement $SO_2F$, m est compris entre 1 et 3 ($1 < m < 3$) et n a la valeur donnée ci-dessus.

L'invention s'étend également au cas où lorsque n est inférieur ou égal à 5, le noyau benzénique comporte en outre un ou plusieurs substituants (par exemple un radical hydrocarboné).

On connaît dans l'art antérieur des procédés de ce type. En effet, G. G. Yakobson et Collaborateurs décrivent dans Chemical Abstracts Vol. 62, 1965 14740 C et Chemical Abstracts Vol. 66, 1967 94741 C la réaction en phase fondue du fluorure de p.chloro benzène sulfonyle, du fluorure de dichloro-2,4 benzène sulfonyle et du fluorure de trichloro-2,4,5 benzène sulfonyle avec du fluorure de potassium à des températures variant entre 240 et 290 °C pendant 25 à 40 heures pour obtenir respectivement le fluorure de p-fluorobenzène sulfonyle, le fluorure de difluoro-2,4 benzène sulfonyle, et le fluorure de chloro-5 difluoro-2,4 benzène sulfonyle avec des rendements respectifs de 60 %, 55 % et 45 %.

Ce type de procédé n'est pas entièrement satisfaisant en vue d'une exploitation industrielle compte tenu principalement des températures élevées qu'il est nécessaire de maintenir pendant des temps réactionnels très longs.

La présente invention pallie les inconvénients de l'art antérieur.

La présente invention a donc pour objet un procédé de préparation de fluorures de fluorobenzène sulfonyle de formule générale :

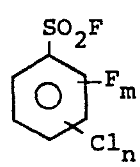

(II)

dans laquelle n est supérieur ou égal à 1 et inférieur ou égal à 5, m est supérieur ou égal à 1 et inférieur ou égal à 3, et F est situé en ortho et/ou para du groupement sulfonyle, par échange d'au moins un atome de chlore par un atome de fluor par réaction d'au moins un fluorure alcalin sur un composé de formule générale :

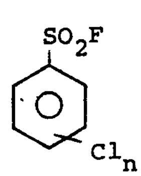

(I)

où n a la signification précédente caractérisé en ce que l'on met en œuvre la réaction dans un solvant

polaire aprotique à une température comprise entre 100 °C et 240 °C.

Le fait que cette réaction puisse être mise en œuvre dans un solvant polaire aprotique est en soi très surprenant. En effet les travaux de Yakobson analysés ci-dessus, montrent que le groupement $SO_2F$, bien qu'électroattracteur, est un groupement très faiblement activant (240-290 °C pendant 25 à 40 heures). Ceci est dû notamment au fait que lors de la réaction entre un composé de formule (II) et un fluorure alcalin, les ions $F^-$ du KF s'échangent d'abord avec l'atome de fluor du groupement $SO_2F$. Il devient nécessaire dans ces conditions d'opérer à des températures très élevées avec des temps de réaction très longs pour pouvoir réaliser l'échange chlore-fluor sur le noyau aromatique. Il est clair pour l'homme de l'art que dans ces conditions de température et de temps de réaction avec les solvants utilisés dans l'art antérieur, notamment les solvants polaires aprotiques, l'introduction de fluor sur les noyaux aromatiques par l'échange chlore-fluor, ne peut être mise en œuvre pour des raisons de stabilité thermique du solvant.

Le caractère surprenant de l'invention tient au fait que les solvants aprotiques polaires permettent à la réaction d'échange chlore-fluor sur le noyau aromatique d'un composé de formule (II) d'avoir lieu dans des conditions de température et de temps de réaction considérablement plus favorables à une exploitation industrielle.

Le solvant est de préférence choisi parmi le groupe comprenant la diméthylformamide, la n-méthylpyrrolidone, le sulfolane, le diméthylsulfoxyde, l'hexaméthyl phosphorotriamide, l'acétonitrile, le propionitrile et le butyronitrile.

On préfère encore plus particulièrement utiliser le sulfolane comme solvant.

On met en œuvre la réaction à une température comprise entre 130 et 220 °C, la stabilité du solvant déterminant la limite supérieure de cette fourchette. On opère de préférence sous pression atmosphérique bien que des pressions inférieures ou supérieures à la pression atmosphérique ne soient pas exclues du domaine de l'invention.

On utilise de préférence une quantité de solvant telle que le nombre de moles du produit de départ, c'est-à-dire du fluorure de chlorobenzène sulfonyle, par litre de solvant est compris entre 0,1 et 6 moles. Encore plus particulièrement, on en utilise entre 0,2 et 3.

On peut utiliser comme fluorure alcalin, le fluorure de sodium, potassium, caesium ou rubidium. Le fluorure de potassium est préféré car c'est le fluorure alcalin le plus intéressant au plan industriel.

La quantité de fluorure alcalin à utiliser dépend évidemment du degré d'échange chlore-fluor que l'on veut atteindre, c'est-à-dire du nombre d'atomes de chlore que l'on veut échanger.

Selon une variante du procédé selon l'invention, on met en œuvre la réaction en présence en outre d'au moins un agent séquestrant de formule générale :

$$N\text{—}[CHR_1\text{—}CHR_2\text{—}O\text{—}(CHR_3\text{—}CHR_4\text{—}O)_p\text{—}R_5]_3 \qquad \text{(III)}$$

dans laquelle p est un nombre entier supérieur ou égal à 0 et inférieur ou égal à 10 ($0 \leq p \leq 10$), $R_1$, $R_2$, $R_3$, $R_4$ identiques ou différents représentent un atome d'hydrogène ou un radical alkyle ayant de 1 à 4 atomes de carbone, $R_5$ représente un radical alkyle ou cycloalkyle ayant de 1 à 12 atomes de carbone, un radical phényle ou un radical de formule $\text{—}C_qH_{2q}\text{—}C_6H_5$ ou $C_qH_{2q+1}\text{—}C_6H_4\text{—}$ q étant compris entre 1 et 12.

L'invention repose sur le fait que l'agent séquestrant de formule (III) forme avec le fluorure alcalin un complexe qui active la réaction et, par suite, améliore le rendement. Cette amélioration de rendement est surtout nuisible dans le cas de composés de départ peu actifs.

Selon un mode de réalisation préférentiel de cette variante du procédé de l'invention, on utilise au moins un agent séquestrant de formule (III) dans laquelle $R_1$, $R_2$, $R_3$, et $R_4$ représentent un atome d'hydrogène ou un radical méthyle, $R_5$ et p ayant la signification précédente.

Parmi ces derniers, on préfère encore plus particulièrement mettre en œuvre les agents séquestrants pour lesquels p est supérieur ou égal à 0 et inférieur ou égal à 3, et pour lesquels $R_5$ représente un radical alkyle ayant de 1 à 4 atomes de carbone.

On peut citer :

— la tris(oxa-3 heptyl) amine de formule :
$N\text{—}(CH_2\text{—}CH_2\text{—}O\text{—}C_4H_9)_3$
— la tris(dioxa-3,6 heptyl) amine de formule :
$N\text{—}(CH_2\text{—}CH_2\text{—}O\text{—}CH_2\text{—}CH_2\text{—}O\text{—}CH_3)_3$
— la tris(trioxa-3,6,9 décyl) amine de formule :
$N\text{—}(CH_2\text{—}CH_2\text{—}O\text{—}CH_2\text{—}CH_2\text{—}O\text{—}CH_2\text{—}CH_2\text{—}O\text{—}CH_3)_3$
— la tris(dioxa-3,6 octyl) amine de formule :
$N\text{—}(CH_2\text{—}CH_2\text{—}O\text{—}CH_2\text{—}CH_2\text{—}O\text{—}C_2H_5)_3$
— la tris(trioxa-3,6,9 undécyl) amine de formule :
$N\text{—}(CH_2\text{—}CH_2\text{—}O\text{—}CH_2\text{—}CH_2\text{—}O\text{—}CH_2\text{—}CH_2\text{—}O\text{—}C_2H_5)_3$
— la tris(dioxa-3,6 nonyl) amine de formule :
$N\text{—}(CH_2\text{—}CH_2\text{—}O\text{—}CH_2\text{—}CH_2\text{—}O\text{—}C_3H_7)_3$
— la tris(trioxa-3,6,9 dodécyl) amine de formule :

$N-(CH_2-CH_2-O-CH_2-CH_2-O-CH_2-CH_2-O-C_3H_7)_3$

— la tris(dioxa-3,6 décyl) amine de formule :

$N-(CH_2-CH_2-O-CH_2-CH_2-O-C_4H_9)_3$

— la tris(trioxa-3,6,9 tridécyl) amine de formule :

$N-(CH_2-CH_2-O-CH_2-CH_2-O-CH_2-CH_2-O-C_4H_9)_3$

On peut encore citer :

— la tris(dioxa-3,6 méthyl-4 heptyl) amine de formule :

$N-(CH_2-CH_2-O-CHCH_3-CH_2-O-CH_3)_3$

— la tris(dioxa-3,6 diméthyl-2,4 heptyl) amine de formule :

$N-(CH_2-CHCH_3-O-CHCH_3-CH_2-O-CH_3)_3$.

Les amines utilisées dans le procédé selon l'invention sont connues en tant que telles dans l'art antérieur. C'est ainsi que le brevet français 1.302.365 cite l'obtention des amines tertiaires $N-(CH_2-CH_2-O-CH_3)_3$ et $N-(CH_2-CH_2-O-CH_2-CH_2-O-CH_3)_3$ comme sous-produits de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1.302.365 précité simultanément aux amines utilisées dans le procédé objet de la présente demande est totalement étranger au domaine de l'invention.

On utilise de préférence, l'agent séquestrant de formule (III) en quantité telle que le rapport molaire de l'agent séquestrant au fluorure alcalin est compris entre 0,001 et 0,2. Ce rapport est, encore plus préférentiellement, compris entre 0,01 et 0,1.

On peut citer comme exemples de composés de départ de formule (I), les composés suivants : le fluorure de chloro-4 benzène sulfonyle, le fluorure de chloro-2 benzène sulfonyle, le fluorure de dichloro-2,5 benzène sulfonyle, le fluorure de dichloro-2,4 benzène sulfonyle, le fluorure de dichloro-3,4 benzène sulfonyle, le fluorure de dichloro-2,6 benzène sulfonyle, le fluorure de dichloro-2,3 benzène sulfonyle, le fluorure de trichloro-2,4,5 benzène sulfonyle, le fluorure de trichloro-2,3,4 benzène sulfonyle, le fluorure de trichloro-2,4,6 benzène sulfonyle, le fluorure de tétrachloro-2,3,4,5 benzène sulfonyle, le fluorure de tétrachloro-2,3,5,6 benzène sulfonyle, le fluorure de tétrachloro-2,3,4,6 benzène sulfonyle et le fluorure de pentachloro benzène sulfonyle.

Ces composés donnent les composés de formule (II) suivants : le fluorure de fluoro-4 benzène sulfonyle, le fluorure de fluoro-2 benzène sulfonyle, le fluorure de fluoro-2 chloro-5 benzène sulfonyle, le fluorure de difluoro-2,4 benzène sulfonyle, le fluorure de fluoro-4 chloro-3 benzène sulfonyle, le fluorure de difluoro-2,6 benzène sulfonyle, le fluorure de fluoro-2 chloro-3 benzène sulfonyle, le fluorure de difluoro-2,4 chloro-5 benzène sulfonyle, le fluorure de difluoro-2,4 chloro-3 benzène sulfonyle, le fluorure de trifluoro-2,4,6 benzène sulfonyle, le fluorure de difluoro-2,4 dichloro-3,5 benzène sulfonyle, le fluorure de difluoro-2,6 dichloro-3,5 benzène sulfonyle, le fluorure de trifluoro-2,4,6 chloro-3 benzène sulfonyle et le fluorure de trifluoro-2,4,6 dichloro-3,5 benzène sulfonyle.

Il est clair pour l'homme de l'art que l'on peut remplacer le fluorure de chlorobenzène sulfonyle de départ par le chlorure correspondant qui se transformera dans les conditions de la réaction dans un premier temps en fluorure.

Les agents séquestrants de formule I utilisés dans le procédé selon l'invention peuvent être préparés par condensation d'un sel de formule :

$$R_5-(O-CHR_4-CHR_3)_p-O-M$$

où $R_3$, $R_4$, $R_5$, et p ont la signification précédente et où M représente un atome de métal alcalin choisi parmi le sodium, le potassium et le lithium, soit sur une amine de formule générale :

$$N-(CHR_1-CHR_2-X)_3$$

dans laquelle $R_1$, et $R_2$ ont la signification précédente et X représente le chlore ou le brome, soit sur le chlorhydrate ou le bromhydrate correspondant.

Le rapport molaire sel de métal alcalin/amine est compris entre 3 et 5.

L'opération de condensation est réalisée à une température comprise entre 100 et 150 °C pendant 1 à 15 heures en présence d'un solvant qui peut être par exemple le chlorobenzène ou de préférence le monoalkyléther d'éthylène glycol de formule :

$$R_5-(O-CHR_4-CHR_3)_p-OH.$$

On opère de préférence de telle sorte qu'on ait une solution contenant de 2 à 5 moles de sel de métal alcalin par litre de solvant.

Le mélange en fin de réaction contient principalement l'amine tertiaire de formule :

$$N-[CHR_1-CHR_2-O-(CHR_3-CHR_4-O)_pR_5]_3$$

mais contient aussi en faible proportion de l'amine secondaire correspondant :

$$HN-[CHR_1-CHR_2-O-(CHR_3-CHR_4-O)_p-R_5]_2$$

et des traces d'amine primaire :

$$H_2N-[CHR_1-CHR_2-O-(CHR_3-CHR_4-O)_p-R_5]$$

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en œuvre de l'invention effectuer une distillation plus poussée du mélange ci-dessus afin d'obtenir une amine tertiaire sensiblement pure.

Les produits de départ sont obtenus de façon classique par l'homme de l'art, par exemple en fluorant le chlorure de chlorobenzène sulfonyle correspondant par échange chlore-fluor. Il en découle que le procédé selon l'invention peut être mis en œuvre également en partant du chlorure de chloro-benzène sulfonyle qui est d'abord transformé en fluorure de chloro-benzène sulfonyle qui, lui-même, est ensuite selon l'invention transformé en fluorure de fluorobenzène sulfonique.

Les produits obtenus selon le procédé de l'invention sont utiles notamment comme intermédiaires de synthèse pour des composés ayant une activité pharmaceutique ou phytosanitaire.

D'autres avantages et caractéristiques de l'invention apparaîtront plus clairement à la lecture des exemples qui vont suivre qui ne sauraient en aucune manière être considérés comme une limitation de l'invention.

## Exemple 1

Préparation du fluorure de parafluorobenzène sulfonyle à partir du fluorure de parachlorobenzène sulfonyle en présence d'agent séquestrant.

Dans un ballon tricol de 250 ml équipé d'un agitateur mécanique, d'un thermomètre et d'un réfrigérant ascendant suivi d'un piège à chlorure de calcium, on introduit 60 g de sulfolane anhydre, 0,1 mole de fluorure de parachlorobenzène sulfonyle (19,5 g), 0,2 mole de KF anhydre (11,6 g) et 0,01 mole de tris(trioxa-3,6,9 décyl) amine (4,5 g).

Le milieu est chauffé à 200 °C pendant 5 heures puis refroidi. Les sels sont filtrés et le mélange soumis à une distillation sous pression réduite.

On obtient ainsi 7 g de fluorure de parafluorobenzène sulfonyle et 10,7 g de fluorure de parachlorobenzène sulfonyle non transformé, soit un rendement de 88 % pour un taux de conversion de 45 %.

## Exemple 2

Préparation du fluorure de chloro-5 difluoro-2,4 benzène sulfonyle à partir du chlorure de trichloro-2,4,5 benzène sulfonyle sans agent séquestrant.

Dans un ballon tricol de 500 ml équipé d'un agitateur mécanique d'un thermomètre et d'un réfrigérant ascendant suivi d'un piège à chlorure de calcium, on introduit 300 ml de sulfolane anhydre, 0,2 mole de chlorure de trichlorobenzène sulfonyle (56 g) et 1 mole de KF anhydre (58 g). Le mélange est chauffé tout d'abord à 100 °C pendant 3 heures au cours desquelles se forme le fluorure de trichloro-2,4, 5 benzène sulfonyle, puis à 160 °C pendant 11 heures. Le milieu est refroidi puis filtré. Par distillation on obtient 21 g de fluorure de chloro-5 difluoro-2,4 benzène sulfonyle.

## Exemple 3

Synthèse du fluorure de difluoro-2,4 benzène sulfonyle à partir du chlorure de dichloro-2,4 benzène sulfonyle.

a) Dans un ballon tricol de 500 ml équipé d'un agitateur mécanique, d'un thermomètre et d'un réfrigérant ascendant suivi d'un piège à chlorure de calcium, on introduit 200 g de sulfolane anhydre, 0,1 mole de chlorure de dichloro-2,4 benzène sulfonyle (M = 245,5) soit 24,6 g et 0,5 mole de KF anhydre soit 29 g.

Le mélange est chauffé tout d'abord à 100 °C pendant 4 heures puis à 170 °C pendant 8 heures. Le rendement en fluorure de difluoro-2,4 benzène sulfonyle est de 34 %.

b) Dans les mêmes conditions opératoires que celles décrites dans l'exemple précédent, on rajoute au milieu réactionnel 0,01 mole de tris(trioxa-3,6,9 décyl) amine (4,6 g). Après 4 heures à 100 °C puis 8 heures à 170 °C, on obtient le fluorure de difluoro-2,4 benzène sulfonyle avec un rendement de 65 %.

Préparation de la tris(trioxa-3,6,9 décyl) amine

Dans un ballon tricol de 1 litre équipé d'un agitateur mécanique, d'un condenseur et d'un thermomètre, on introduit 600 g d'éther monométhylique du diéthylène glycol (dioxa-3,6 heptanol-1) soit 5 moles puis 23 g de sodium (1 mole) par petites fractions afin de former le dioxa-3,6 heptanolate de sodium.

Lorsque le sodium est totalement transformé, on ajoute alors 51,8 g de chlorhydrate de la tris(chloro-2 éthyl) amine (soit 0,215 mole). Le mélange est chauffé à 130 °C pendant 8 heures sous agitation puis refroidi et l'excès d'alcoolate de sodium neutralisé par une solution aqueuse d'acide chlorhydrique à 10 %. Le dioxa-3,6 heptanol-1 est éliminé par distillation à 130 °C sous 20 mmHg. Le mélange obtenu est filtré afin d'éliminer le chlorure de sodium puis le produit est distillé. On obtient ainsi 83 g de tris(trioxa-3,6,9 décyl) amine qui distille à 189 °C sous 0,1 mmHg.

**Revendications**

1. Procédé de préparation de fluorures de fluorobenzène sulfonyle de formule générale :

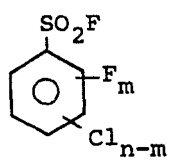

(II)

dans laquelle n est supérieur ou égal à 1 et inférieur ou égal à 5, m est supérieur ou égal à 1 et inférieur ou égal à 3, et F est situé en ortho et ou para du groupement sulfonyle, par échange d'au moins un atome de chlore par un atome de fluor par réaction du fluorure alcalin sur un composé de formule générale :

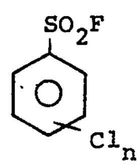

(I)

où n a la signification précédente, caractérisé en ce qu'on met en œuvre la réaction dans un solvant polaire aprotique à une température comprise entre 100 °C et 240 °C.

2. Procédé selon la revendication 1 caractérisé en ce que le solvant aprotique polaire est choisi parmi le groupe comprenant la diméthylformamide, la n-méthylpyrrolidone, le sulfolane, le diméthylsulfoxyde et l'hexaméthyl phosphoramide.

3. Procédé selon la revendication 1 caractérisé en ce que le solvant aprotique polaire est le sulfolane.

4. Procédé selon la revendication 1 caractérisé en ce que l'on met en œuvre la réaction à une température comprise entre 130 et 220 °C.

5. Procédé selon l'une quelconque des revendications 1, 2 et 3, caractérisé en ce que l'on utilise une quantité de solvant telle que le nombre de moles de composé de formule (I) par litre de solvant est compris entre 0,1 et 6 moles/litre.

6. Procédé selon la revendication 5 caractérisé en ce que le nombre de moles de composé de formule (I) par litre de solvant est compris entre 0,2 et 3 moles/litre.

7. Procédé selon l'une quelconque des revendications précédentes, caractérisé en ce que l'on met en œuvre la réaction en présence en outre d'au moins un agent séquestrant de formule générale :

$$N—[CHR_1—CHR_2—O—(CHR_3—CHR_4—O)_p—R_5]_3 \quad (III)$$

dans laquelle p est un nombre entier supérieur ou égal à 0 et inférieur ou égal à 10 ($0 \leq p \leq 10$), $R_1$, $R_2$, $R_3$, $R_4$ identiques ou différents représentent un atome d'hydrogène ou un radical alkyle ayant de 1 à 4 atomes de carbone, $R_5$ représente un radical alkyle ou cycloalkyle ayant de 1 à 12 atomes de carbone, un radical phényle ou un radical de formule : $—C_qH_{2q}—C_6H_5$ ou $C_qH_{2q+1}—C_6H_4—$, q étant compris entre 1 et 12.

8. Procédé selon la revendication 7 caractérisé en ce que dans la formule (III) de l'agent séquestrant, $R_1$, $R_2$, $R_3$ et $R_4$ identiques ou différents représentent un atome d'hydrogène ou un radical méthyle.

9. Procédé selon la revendication 7 caractérisé en ce que dans la formule (III) de l'agent séquestrant,

p est supérieur ou égal à 0 et inférieur ou égal à 5.

10. Procédé selon la revendication 7 caractérisé en ce que dans la formule (III) de l'agent séquestrant, $R_5$ représente un radical alkyle ayant de 1 à 4 atomes de carbone.

11. Procédé selon la revendication 7, caractérisé en ce que dans la formule (III) de l'agent séquestrant, $R_1$, $R_2$, $R_3$ et $R_4$ identiques ou différents représentent un atome d'hydrogène ou un radical méthyle, p est un nombre entier supérieur ou égal à 0 et inférieur ou égal à 3 et $R_5$ représente un radical alkyle ayant de 1 à 4 atomes de carbone.

12. Procédé selon la revendication 7 caractérisé en ce que l'agent séquestrant de formule (III) est la tris(trioxa-3,6,9 décyl) amine de formule :

$$N-(CH_2-CH_2-O-CH_2-CH_2-O-CH_2-CH_2-O-CH_3)_3$$

## Claims

1. Process for the preparation of fluorobenzenesulphonyl fluorides of the general formula :

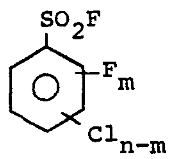

(II)

in which n is greater than or equal to 1 and less than or equal to 5, m is greater than or equal to 1 and less than or equal to 3, and F is located in the ortho and/or para positions relative to the sulphonyl group, by means of the exchange of at least one chlorine atom by a fluorine atom by reaction of an alkali metal fluoride with a compound of the general formula :

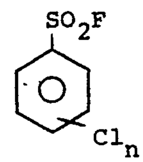

(I)

in which n has the above meaning, characterised in that the reaction is carried out in a polar aprotic solvent at a temperature of between 100 °C and 240 °C.

2. Process according to Claim 1, characterised in that the polar aprotic solvent is chosen from amongst the group comprising dimethylformamide, N-methylpyrrolidone, sulpholan, dimethyl sulphoxide and hexamethylphosphoramide.

3. Process according to Claim 1, characterised in that the polar aprotic solvent is sulpholan.

4. Process according to Claim 1, characterised in that the reaction is carried out at a temperature of between 130 and 220 °C.

5. Process according to any one of Claims 1, 2 and 3, characterised in that the amount of solvent used is such that the number of mols of compound of the formula (I) per litre of solvent is between 0.1 and 6 mols/litre.

6. Process according to Claim 5, characterised in that the number of mols of compound of the formula (I) per litre of solvent is between 0.2 and 3 mols/litre.

7. Process according to any one of the preceding Claims, characterised in that the reaction is also carried out in the presence of at least one sequestering agent of the general formula :

$$N-[CHR_1-CHR_2-O-(CHR_3-CHR_4-O)_p-R_5]_3 \quad (III)$$

in which p is an integer which is greater than or equal to 0 and less than or equal to 10 ($0 \leq p \leq 10$), $R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical of the formula : $-C_qH_{2q}-C_6H_5$ or $C_qH_{2q+1}-C_6H_4-$, q being between 1 and 12.

8. Process according to Claim 7, charactetised in that, in the formula (III) of the sequestering agent, $R_1$, $R_2$, $R_3$ and $R_4$, which are identical or different represent a hydrogen atom or a methyl radical.

9. Process according to Claim 7, characterised in that, in the formula (III) of the sequestering agent, p is greater than or equal to 0 and less than or equal to 5.

10. Process according to Claim 7, characterised in that, in the formula (III) of the sequestering agent, $R_5$ represents an alkyl radical having from 1 to 4 carbon atoms.

11. Process according to Claim 7, characterised in that, in the formula (III) of the sequestering agent, $R_1$, $R_2$, $R_3$ and $R_4$, which are identical or different, represent a hydrogen atom or a methyl radical, p is an integer which is greater than or equal to 0 and less than or equal to 3, and $R_5$ represents an alkyl radical having from 1 to 4 carbon atoms.

12. Process according to Claim 7, characterised in that the sequestering agent of the formula (III) is tris-(3,6,9-trioxadecyl)-amine of the formula :

$$N-(CH_2-CH_2-O-CH_2-CH_2-O-CH_2-CH_2-O-CH_3)_3.$$

**Ansprüche**

1. Verfahren zur Herstellung von Fluorbenzolsulfonylfluoriden der allgemeinen Formel,

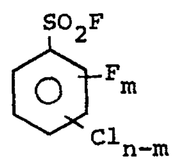

(II)

in der n größer oder gleich 1 und kleiner oder gleich 5, m größer oder gleich 1 und kleiner oder gleich 3 ist und F in Ortho- oder Para-Stellung zur Sulfonylgruppe steht, durch Austausch wenigstens eines Chloratoms durch ein Fluoratom infolge der Einwirkung von Alkalifluorid auf eine Verbindung der allgemeinen Formel

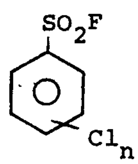

(I)

in der n die obige Bedeutung hat, dadurch gekennzeichnet, daß man die Reaktion in einem aprotischen polaren Lösungsmittel bei einer Temperatur zwischen 100 und 240 °C durchführt.

2. Verfahren nach Anspruch 1, dadurch gekennzeichnet, daß das aprotische polare Lösungsmittel aus der Gruppe von Dimethylformamid, N-Methylpyrrolidon, Sulfolan, Dimethylsulfoxid und Hexamethylphosphoramid gewählt ist.

3. Verfahren nach Anspruch 1, dadurch gekennzeichnet, daß das aprotische polare Lösungsmittel Sulfolan ist.

4. Verfahren nach Anspruch 1, dadurch gekennzeichnet, daß man die Reaktion bei einer Temperatur zwischen 130 und 220 °C durchführt.

5. Verfahren nach einem der Ansprüche 1, 2 und 3, dadurch gekennzeichnet, daß man das Lösungsmittel in einer solchen Menge verwendet, daß die Anzahl der Mole der Verbindung der Formel (I) pro Liter Lösungsmittel 0,1 bis 6 Mol/l beträgt.

6. Verfahren nach Anspruch 5, dadurch gekennzeichnet, daß die Anzahl der Mole der Verbindung der Formel (I) pro Liter Lösungsmittel 0,2 bis 3 Mol/Liter beträgt.

7. Verfahren nach einem der vorangegangenen Ansprüche dadurch gekennzeichnet, daß man die Reaktion zusätzlich in Gegenwart mindestens eines Sequestrierungsmittels der allgemeinen Formel

$$N-[CHR_1-CHR_2-O-(CHR_3-CHR_4-O)_p-R_5]_3 \quad (III)$$

durchführt, in der p eine ganze Zahl größer oder gleich 0 und kleiner oder gleich 10 ($0 \leq p \leq 10$) ist, $R_1$, $R_2$, $R_3$, $R_4$ gleich oder verschieden ein Wasserstoffatom oder einen Alkylrest mit 1 bis 4 Kohlenstoffatomen, $R_5$ einen Alkyl- oder Cycloalkylrest mit 1 bis 12 Kohlenstoffatomen oder einen Rest der Formel $-C_qH_{2q}-C_6H_5$ oder $C_qH_{2q+1}-C_6H_4-$ bedeuten, wobei q 1 bis 12 ist.

8. Verfahren nach Anspruch 7, dadurch gekennzeichnet, daß in der Formel (III) des Sequestrierungsmittels $R_1$, $R_2$, $R_3$ und $R_4$ gleich oder verschieden ein Wasserstoffatom oder einen Methylrest bedeuten.

9. Verfahren nach Anspruch 7, dadurch gekennzeichnet, daß in der Formel (III) des Sequestrierungsmittels p größer oder gleich 0 und kleiner oder gleich 5 ist.

10. Verfahren nach Anspruch 7, dadurch gekennzeichnet, daß in der Formel (III) des Sequestrierungsmittels $R_5$ einen Alkylrest mit 1 bis 4 Kohlenstoffatomen bedeutet.

11. Verfahren nach Anspruch 7, dadurch gekennzeichnet, daß in der Formel (III) des Sequestrierungsmittels $R_1$, $R_2$, $R_3$ und $R_4$ gleich oder verschieden ein Wasserstoffatom oder einen Methylrest bedeuden, p eine ganze Zahl größer oder gleich 0 und kleiner oder gleich 3 ist und $R_5$ einen Alkylrest mit

1 bis 4 Kohlenstoffatomen bedeutet.

12. Verfahren nach Anspruch 7, dadurch gekennzeichnet, daß das Sequestrierungsmittel der Formel (III) Tris(3,6,9-trioxadecyl) amin der Formel

$$N{-}(CH_2{-}CH_2{-}O{-}CH_2{-}CH_2{-}O{-}CH_2{-}CH_2{-}O{-}CH_3)_3$$

ist.